# EUROPÄISCHE PATENTANMELDUNG

(11) **EP 1 813 251 A2**
(43) Veröffentlichungstag der Anmeldung: **01.08.2007**
(21) Anmeldenummer: 07000844.6
(22) Anmeldetag: 17.01.2007
(51) Int. Cl.: A61K 8/02, A61K 8/06, A61K 8/37, A61K 8/34, A61Q 19/00, A61Q 19/10, A61Q 17/04, A61Q 1/00, A61Q 15/00, C11D 1/83, C11D 17/06

(54) **Kaltherstellbare, niedrigviskose und langzeitstabile kosmetische Emulsionen**

(30) Priorität: 30.01.2006 DE 10604353
(71) Anmelder: Goldschmidt GmbH, 45127 Essen (DE)
(72) Erfinder: Hameyer, Peter, 45138 Essen (DE); Meyer, Jürgen, Dr., 48143 Münster (DE); Polak, Gabriele, 58099 Hagen (DE)

(57) **Zusammenfassung**

Gegenstand der Erfindung sind PEG-freie, kaltherstellbare langzeitstabile, niedrigviskose, feinteilige Öl-in-Wasser-Emulsionen, deren Herstellung aus vorzugsweise klaren Ölphasen bzw. über vorzugsweise klare bis transparente mikroemulsionsartige Konzentrate, die entsprechenden Ölphasen bzw. mikroemulsionsartigen Konzentrate sowie die Verwendung der erfindungsgemäßen Emulsionen zur Herstellung von kosmetischen, dermatologischen, pharmazeutischen oder technischen Zubereitungen, insbesondere zur Herstellung von Tränkemulsionen für Feuchttücher (Wet Wipes) oder für sprühbare Pflegeemulsionen.

## Beschreibung

Gegenstand der Erfindung sind kaltherstellbare langzeitstabile, niedrigviskose, feinteilige Öl-in-Wasser-Emulsionen, deren Herstellung aus vorzugsweise klaren Ölphasen bzw. über vorzugsweise klare bis transparente mikroemulsionsartige Konzentrate, die entsprechenden Ölphasen bzw. mikroemulsionsartigen Konzentrate sowie die Verwendung der erfindungsgemäßen Emulsionen zur Herstellung von kosmetischen, dermatologischen oder pharmazeutischen Zubereitungen sowie zur Herstellung von Reinigungs- und Pflegeemulsionen für Haushalt und Industrie, insbesondere zur Herstellung von Tränkemulsionen für Feuchttücher (Wet Wipes) oder für sprühbare Pflegeemulsionen.

Die erfindungsgemäßen feinteiligen Öl-in-Wasser-Emulsionen sind dabei vorzugsweise frei von polyethylenglykolhaltigen Substanzen ("PEG-frei") und enthalten eine Emulgatormischung, bestehend aus einer primären nichtionischen Emulgatorkomponente, vorzugsweise Polyolpartialestern, und einer sekundären neutralisierbaren Emulgatorkomponente, darüber hinaus grenzflächenaktive Cotenside, vorzugsweise aromatische Cotenside mit konservierenden Eigenschaften, sowie darüber hinaus Öle, vorzugsweise Ester und/oder Etheröle und gegebenenfalls weitere übliche Hilfs- und Zusatzstoffe.

Emulsionen stellen einen wichtigen Produkttyp im Bereich kosmetischer, dermatologischer und/oder pharmazeutischer Zubereitungen dar. Kosmetische Zubereitungen werden im Wesentlichen zur Hautpflege benutzt. Hautpflege im kosmetischen Sinn ist in erster Linie, dass die natürliche Funktion der Haut als Barriere gegen Umwelteinflüsse (beispielsweise Schmutz, Chemikalien, Mikroorganismen) und gegen den Verlust von körpereigenen Stoffen (beispielsweise Wasser, natürliche Fette, Elektrolyte) gestärkt und/oder wiederhergestellt wird.

Ziel der Hautpflege ist ferner, den durch tägliches Waschen verursachten Fett- und Wasserverlust der Haut auszugleichen und die Weichheit und Glätte der Haut zu erhalten bzw. wieder herzustellen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind schützen und die Hautalterung verzögern.

Pharmazeutische topische Zusammensetzungen enthalten in der Regel ein oder mehrere Medikamente in wirksamer Konzentration. Der Einfachheit halber wird zur eindeutigen Unterscheidung zwischen kosmetischer und medizinischer Anwendung und entsprechenden Produkten auf die gesetzlichen Bestimmungen der Bundesrepublik Deutschland verwiesen (beispielsweise Kosmetikverordnung, Lebensmittel- und Arzneimittelgesetz).

In den letzten Jahren gewannen kosmetische Feuchttücher aufgrund ihrer überaus einfachen und bequemen Verwendbarkeit zunehmende Bedeutung. Zunächst waren nahezu ausschließlich Feuchttücher für Reinigungszwecke auf dem Kosmetikmarkt vertreten, die hauptsächlich wässrige, tensidische Tränklösungen enthielten. Mehr und mehr erschienen aber in der letzten Zeit auch Pflegeprodukte auf dem Markt, die auf Tränkemulsionen basieren und somit zusätzlich eine pflegende Ölkomponente enthalten.

Die meisten dieser kosmetischen Feuchttücher für Körper- und Gesichtspflege sind dabei mit Emulsionen getränkt, die nach der PIT-Emulgiermethode ( K. Shinoda, H. Kunieda, Phase properties of emulsions: PIT and HLB, Encycl. of Emulsion Technology, 337-367 (1), 1983 oder Th. Förster, F. Schambil, W. von Rybinski, J. Disp. Sci. And Technology, 13(2), 183-93 (1992)) hergestellt wurden.

Die PIT-Methode macht sich die Tatsache zunutze, dass in einer Öl-in-Wasser(O/W)-Emulsion, die durch nichtionische, Polyethylenglykol enthaltende Emulgatoren ("PEG-haltige Emulgatoren") stabilisiert ist, durch Temperaturerhöhung eine Phaseninversion zu einer Wasser-in-Öl(W/O)-Emulsion induziert werden kann (Phaseninversion; PIT: Phaseninversionstemperatur).

Da in diesem Phaseninversionsbereich die Grenzflächenspannung Wasser-Öl extrem gering ist, können so nach Abkühlung äußerst feinteilige Öl-in-Wasser-Emulsionen erhalten werden. Dazu ist allerdings notwendig, dass für jedes zu emulgierende System die einzelnen Komponenten der Emulsionen genau aufeinander abgestimmt werden. Das heißt, Emulgatormischungen und Emulgatorkonzentration müssen für unterschiedliche Ölphasen "maßgeschneidert" werden.

Die so hergestellten feinteiligen und niedrigviskosen Emulsionen besitzen eine ausgezeichnete Langzeitstabilität und eignen sich somit gut als Tränklösungen für Feuchttücher. Derartige Systeme sind beispielsweise in EP-B-1 268 740 oder in WO-A-00/04230 beschrieben.

Ein grundlegendes Erfordernis zur Anwendung der PIT-Emulgiertechnik ist wie beschrieben die Notwendigkeit, dass die gesamte Emulsion auf Temperaturen oberhalb der Phaseninversionstemperatur erhitzt werden und im Anschluss daran auch wieder abgekühlt werden muss.

In den heutigen Zeiten, in denen Prozessabläufe optimiert und Energiekosten begrenzt werden müssen, bedeutet dies einen deutlichen Nachteil gegenüber Systemen, die diese Aufheiz-/Abkühlkurve nicht durchlaufen müssen. Aus diesem Grund wären feinteilige, langzeitstabile Emulsionen vorteilhaft, die bei Raumtemperatur hergestellt werden können ("Kaltherstellung") ohne eine zusätzliche Aufheiz-/Abkühlkurve durchlaufen zu müssen.

Nachteilig bei Tränklösungen für Feuchttücher auf Basis von PIT-Emulsionen ist außerdem, dass sie auf der Verwendung PEG-haltiger Emulgatoren beruht. Im Zuge von möglichst natürlichen Kosmetikformulierungen ist ein wichtiges Ziel der Kosmetikforschung, auf Polyethylenglykol ("PEG") enthaltende Emulgatoren verzichten zu können. Daher besteht eine verstärkte Suche nach PEG-freien Alternativlösungen.

Weiterhin ist bekannt, dass ethoxylierte Emulgatoren ein eher wässriges Hautgefühl vermitteln, was durch den Einsatz von beispielsweise Polyglycerinestern sensorisch verbessert werden kann.

So beschreibt WO-A-02/056841 PEG-freie Tränkemulsionen für kosmetische Feuchttücher auf Basis von Polyolpoly-12-hydroxystearaten und Alkylglykosiden. Der Einsatz dieser Emulgatormischungen führt zu einem verbesserten Weichgriff der damit getränkten Papierprodukte und führt auch ansonsten zu verbesserten sensorischen Eigenschaften bei Verwendung der damit hergestellten Feuchttücher. Bei solchen Emulgatorkömbinationen ist es allerdings in der Regel schwierig, eine gute Langzeitstabilität der Tränkemulsionen in Kombination mit einer ausreichenden Konservierung zu erreichen.

Insbesondere bei der Herstellung von Feuchttüchern ist eine ausreichende Konservierung der Tränklösungen zwingend erforderlich, um Verkeimungen vorzubeugen. Dabei muss die Konservierung ausreichend sein, um sowohl die Tränklösungen selbst, sowie schließlich auch die getränkten Feuchttücher langzeitig gegenüber Verkeimungen zu schützen.

Als bevorzugte Konservierungsmischungen werden dabei typischerweise Mischungen von Alkylparabenestern und Phenoxyethanol eingesetzt, wie sie etwa unter den Handelsnamen Euxyl^{®} K 300 (Schülke & Mayr) oder Phenonip^{®} (Clariant) kommerziell erhältlich sind.

Die beschriebenen hohen Anforderungen bzgl. einer sicheren Konservierung von Tränklösung und Feuchttüchern machen es dabei notwendig, dass in der Regel relativ große Mengen dieser Alkylparabenester/Phenoxyethanol-Mischungen in den fertigen Tränklösungen eingesetzt werden müssen (0,5 bis 1,0 Gew.-%). Idealerweise sollte bei der Herstellung von Emulsionskonzentraten bereits die gesamte Menge an Konservierungsmittel eingearbeitet sein. Dies erlaubt, dass die gewünschte Einsatzkonzentration der Tränklösung anschließend einfach durch Verdünnung mit Wasser eingestellt werden kann.

Es ist bekannt, dass der Einsatz dieser Alkylparabenester/Phenoxyethanol-Mischungen emulsionsbelastenden Einfluss hat, da diese Verbindungen sehr grenzflächenaktiv sind und mit Emulgatormolekülen um einen Platz an der Grenzfläche Öl-Wasser konkurrieren. Aufgrund des grenzflächenaktiven Charakters dieser Konservierungsmittelmischungen kann man sie daher auch als aromatische Cotenside mit konservierenden Eigenschaften beschreiben. Verstärkt wird dieser emulsionsbelastende Effekt im Fall von Tränkemulsionen für Feuchttücher in der Regel durch die erforderlichen hohen Mengen dieser Konservierungsmittel und die geringen Viskositäten der Tränklösungen.

Zusammenfassend ist es daher festzustellen, dass mit den im Stand der Technik beschriebenen Emulgatoren oder Emulgatorkombinationen nicht möglich ist, kalt herstellbare, ausreichend konservierte, niedrigviskose, feinteilige und langzeitstabile Emulsionen herzustellen, wie sie typischerweise für Tränkemulsionen oder sprühbare Lotionen verwendet werden.

Eine Aufgabe der vorliegenden Erfindung war es daher niedrigviskose, feinteilige und langzeitstabile Emulsionen herzustellen, wie sie typischerweise für Tränkemulsionen oder sprühbare Lotionen verwendet werden, d.h. die gleichzeitig
- bei Raumtemperatur hergestellt werden können und
- frei von ethoxylierten Bestandteilen sind und
- die darüber hinaus eine ausreichende Menge konservierend wirkender Verbindungen enthalten.

Überraschenderweise wurde nun gefunden, dass niedrigviskose, feinteilige und langzeitstabile Öl-in-Wasser-Emulsionen, die hervorragend zum Einsatz als Tränkemulsionen oder in sprühbaren Systemen geeignet sind, bei Raumtemperatur herstellbar sind, wenn eine geeignete Kombination von Emulgatoren auf Basis von Polyolpartialestern und neutralsierbaren Säurepartialestern zusammen mit Ölen, vorzugsweise Ester und/oder Etheröle und Cotenside, vorzugsweise aromatische Cotenside mit konservierenden Eigenschaften, eingesetzt werden.

Diese Emulsionen zeichnen sich neben ihrer einfachen Herstellung und ihrem feinen Dispersionsgrad dadurch aus, dass sie im Wesentlichen frei von ethoxylierten Inhaltsstoffen sind ("PEG-freie" Emulsionssysteme). Die mit Hilfe dieser Tränklösungen hergestellten Feuchttücher zeichnen sich darüber hinaus durch überaus angenehme sensorische Eigenschaften aus.

Mit den erfindungsgemäßen Öl-in-Wasser-Emulsionen sind erstmals PEG-freie, bei Raumtemperatur herstellbare, niedrigviskose und feinteilige Emulsionen verfügbar, die durch den erfindungsgemäß bevorzugten Einsatz konservieraktiver aromatischer Cotenside gleichzeitig ausreichend konserviert und langzeitstabil sind und sich somit insbesondere für den Einsatz als Tränkemulsion für Feuchttücher eignen.

Ein Gegenstand der Erfindung sind daher langzeitstabile, niedrigviskose, feinteilige Öl-in-Wasser-Emulsionen, enthaltend:
A) eine Emulgatormischung bestehend aus:
   a) mindestens einem nichtionischen primären Emulgator und
   b) mindestens einem ein oder mehrere, Säurefunktionen enthaltenden, sekundären Emulgator, dessen Säurefunktion gegebenenfalls ganz oder teilweise neutralisiert sein kann und
B) ein oder mehrere Cotenside und
C) ein oder mehrere Öle und gegebenenfalls
D) ein oder mehrere polare Lösungsvermittler und gegebenfalls
E) übliche Hilfs- und Zusatzstoffe,
mit der Maßgabe, dass der Wasserphasengehalt der Emulsionen ≥ 80 Gew.-% bezogen auf Gesamtemulsion liegt.

Erfindungsgemäß bevorzugte Emulsionen sind niedrigviskos, feinteilig und langzeitstabil.

Unter niedrigviskos versteht man dabei eine Viskosität, die ein Versprühen der Emulsionen mit üblichen Apparaturen ermöglicht. In der Regel handelt es sich dabei um Emulsionsviskositäten ≤ 4000 mPas (Brookfield RVT, Spindle 4, 10 rpm (20°C)), vorzugsweise ≤ 2500 mPas, besonders bevorzugt s 1000 mPas. Höhere Viskositäten sind einstellbar, jedoch erfindungsgemäß nicht bevorzugt.

Unter feinteilig versteht man dabei einen mittleren Radius der Emulsionstropfen von ≥20-≤500 nm, bevorzugt von ≥30-≤200 nm und besonders bevorzugt von ≥40-≤120 nm.

Unter langzeitstabil versteht man dabei, dass die erfindungsgemäßen Emulsionen drei Monate bei Raumtemperatur und 1 Monat bei 40°C ohne irreversible Aufrahmung oder sonstigen Anzeichen für Instabilität gelagert werden können.

Die erfindungsgemäßen Öl-in-Wasser-Emulsionen haben üblicherweise einen Wasserphasengehalt von ≥70-≤99 Gew.-%, vorzugsweise von ≥80-≤97 Gew.-%.

Zur Wasserphase rechnet man dabei alle Substanzen einer Rezeptur, die aufgrund ihres hydrophilen Charakters dieser Phase beigemischt bzw. in ihr gelöst oder dispergiert werden können. Bezogen auf die erfindungsgemäßen Öl-in-Wasser-Emulsionen gehören somit in jedem Fall Wasser oder etwaige Bestandteile wie Glykole, Polyalkylenglykole, Glycerin, Polyglycerine, Alkohole, wasserlösliche Polymere oder Wirkstoffe zur Wasserphase.

In allen Gegenständen der Erfindung werden die Emulgatormischung (A), die Cotenside (B) und die Öle (C) dabei bevorzugt in Gewichtsanteilen (bezogen auf diese drei Komponenten) von (A) ≥10-≤30 /(B) ≥3-≤20 /(C) ≥50-≤87 und besonders bevorzugt in Gewichtsanteilen von ≥20-≤25 / ≥5-≤ 15 / ≥60-≤75 eingesetzt, wobei sich die Emulgatormischung (A) zusammensetzt aus ≥75-≤99,5 Gew.-% nichtionischen primären Emulgator (a) und ≥0,5-≤25 Gew.-% Säuregruppen enthaltenden sekundären Emulgator (b).

Für die Emulgatormischung (A) werden für die nichtionischen primären Emulgatoren (a) erfindungsgemäß bevorzugt Polyolpartialestern verwendet, ausgewählt aus mindestens einer der Gruppen:
a1) Glycerin- und Polyglycerinpartialester, vorzugsweise hergestellt durch Veresterung von aliphatischen, linearen oder verzweigten, ggf. ungesättigten und/oder hydroxyfunktionalisierten Carbonsäuren mit einer Kettenlänge von ≥6 bis ≤22 C-Atomen mit Glycerin, Polyglycerinen oder Gemischen aus beiden,
a2) Sorbitan oder Sorbitolpartialester, vorzugsweise hergestellt durch Veresterung von aliphatischen, linearen oder verzweigten, ggf. ungesättigten und/oder hydroxyfunktionalisierten Carbonsäuren mit einer Kettenlänge von ≥6 bis ≤22 C-Atomen mit Sorbitol,
a3) Kohlenhydratester, bevorzugt Glykosid- oder Sucroseester, vorzugsweise hergestellt durch Veresterung von aliphatischen, linearen oder verzweigten, ggf. ungesättigten und/oder hydroxyfunktionalisierten Carbonsäuren mit einer Kettenlänge von ≥6 bis ≤22 C-Atomen mit Mono- oder Polysacchariden, sowie gegebenenfalls darüber hinaus,
a4) (Alkylpoly)Glykoside, vorzugsweise hergestellt durch Umsetzung von aliphatischen, linearen oder verzweigten, ggf. ungesättigten und/oder zusätzlich hydroxyfunktionalisierten Alkoholen mit einer Kettenlänge von ≥6 bis ≤22 C-Atomen mit Mono- oder Polysacchariden
oder Mischungen daraus.

Üblicherweise besteht der Hauptteil der nichtionischen primären Emulgatorkomponente (a) aus Polyglycerinestern, denen vorzugsweise Sorbitanester in einer Menge von ≥0-≤75 Gew.-%, vorzugsweise ≥0-≤50 Gew.-%, besonders bevorzugt ≥0-≤25 Gew.-%, bezogen auf gesamte primäre Emulgatorkomponente (a), beigemischt werden.

Bevorzugt sind dabei Polyglycerin- und Sorbitanpartialester, die als hydrophobe Anteile Fettsäurereste mit einer Kettenlänge von ≥10 bis ≤18 C-Atomen enthalten.

Ganz besonders bevorzugt ist eine Kombination aus Polyglyceryllauraten und Sorbitanlauraten.

Für die Emulgatormischung (A) werden für die sekundäre Emulgatorkomponente (b) vorzugsweise Verbindungen ausgewählt aus mindestens einer der Gruppen:
b1) Gegebenenfalls Hydroxylgruppen enthaltende Di- oder Polycarboxylate, sulfatierte, sulfonierte oder phosphatierte Carboxylate, Malonate, Malate, Succinate, Sulfosuccinate, Citrate, Tartrate, bei denen die Säuregruppen teilweise verestert wurden mit aliphatischen oder aromatischen, linearen oder verzweigten, ggf. ungesättigten und/oder hydroxyfunktionalisierten Alkoholen mit einer Kettenlänge von ≥6 bis ≤22 C-Atomen,
b2) Gegebenenfalls Hydroxylgruppen enthaltende Di- oder Polycarboxylate, sulfatierte oder sulfonierte oder phosphatierte Carboxylate, Malonate, Malate, Succinate, Sulfosuccinate, Citrate, Tartrate, bei denen die Säuregruppen teilweise verestert wurden mit Polyolen, Polyolpartialestern, bevorzugt aus Glycerin, Polyglycerin und/oder Sorbitol mit aliphatischen oder aromatischen, linearen oder verzweigten, ggf. ungesättigten und/oder hydroxyfunktionalisierten Carbonsäuren mit einer Kettenlänge von ≥6 bis ≤ 22 C-Atomen,
b3) Polyole, bevorzugt Glycerin, Polyglycerin und Sorbitol, welche teilverestert sind mit aliphatischen oder aromatischen, linearen oder verzweigten, ggf. ungesättigten und/oder hydroxyfunktionalisierten Mono-, Di- oder Polycarbonsäuren mit einer Kettenlänge von ≥2 bis ≤22 C-Atomen, mit der Maßgabe, dass im Molkekül freie, neutralisierbare Säuregruppen vorliegen,
b4) Hydroxyfunktionelle Mono-, Di- oder Polycarbonsäuren, deren Hydroxylgruppen zumindest partiell mit aliphatischen, linearen oder verzweigten, ggf. ungesättigten und/oder hydroxyfunktionalisierten Carbonsäuren mit einer Kettenlänge von ≥6 bis ≤22 C-Atomen umgesetzt wurden,
b5) N-Acylaminosäuren, wie Sarcosinate, Glutamate, Asparaginate, enthaltend einen aliphatischen oder aromatischen, linearen oder verzweigten, ggf. ungesättigten und/oder hydroxyfunktionalisierten Rest mit einer Kettenlänge von ≥6 bis ≤22 C-Atomen,
b6) Carboxylate, Sulfate, Sulfonate, Phosphonate oder Phosphate, enthaltend einen aliphatischen oder aromatischen, linearen oder verzweigten, ggf. ungesättigten und/oder hydroxyfunktionalisierten Acylrest mit einer Kettenlänge von ≥6 bis ≤22 C-Atomen
oder Mischungen daraus.

Die Emulgatoren des Typs (b) liegen in der Emulgatorformulierung vorzugsweise in zumindest teilweise neutralisierter Form vor. Sie werden vorteilhafterweise bereits als (teil)neutralisierte Komponenten eingesetzt. Falls gewünscht, kann der Neutralisationsschritt aber auch in einem geeigneten späteren Verfahrensschritt erfolgen, wobei zur Neutralisation bevorzugt Basen verwendet werden, die zu anionaktiven Emulgatoren mit ein- oder zweiwertigen kationischen Gegenionen führen. Besonders bevorzugt als Gegenionen sind dabei Natrium und Kalium.

Bevorzugt werden neutralisierte Zitronensäurepartialester eingesetzt, deren hydrophobe Reste jeweils ≥10 bis ≤18 C-Atome enthalten.

Ganz besonders bevorzugt sind dabei die Partialester aus Zitronensäure und Laurylalkohol bzw. aus Zitronensäure und Glyceryl Mono- oder Dilauraten, sowie weiterhin Partialester aus Zitronensäure und Oleylalkohol bzw. Zitronensäure und Glyceryl Mono- oder Dioleaten.

Unter Cotensiden im Sinne der vorliegenden Erfindung werden insbesondere solche Verbindungen verstanden, die sich durch Grenzflächenaktivität auszeichnen, was sich in der Absenkung von Grenzflächenspannungen oder der Einlagerung in Grenzflächenfilme äußern kann, ohne dass diese Substanzen allerdings für sich alleine genommen die für Tenside typische Aggregation zu mizellaren Strukturen in Wasser oder die für Emulgatoren typische Stabilisierung von Emulsionströpfchen zeigen würden.

Unter Cotensiden im Sinne der vorliegenden Erfindung werden außerdem oder alternativ insbesondere solche Verbindungen verstanden, welche einen HLB-Wert nach Griffins zwischen ≥4 bis ≤10 aufweisen. Besonders bevorzugt zeichnen sich diese Cotenside durch einen Octanol-Wasser Partitionskoeffizienten log P oder log K_{ow} aus, der zwischen 1 und 2 liegt. Der Octanol-Wasser Verteilungs-koeffizient errechnet sich aus dem dekadischen Logarithmus des Quotienten der im Gleichgewicht bei Raumtemperatur gelösten Menge eines Stoffes in Octanol und in Wasser (siehe: Ullmann's Encyclopedia of Industrial Chemistry, Volume B 7, (Volume Editor: E. Weise), 5th edition, VCH, Weinheim 1995, S. 78.

Vorteilhafterweise handelt es sich bei den erfindungsgemäßen Cotensiden um nichtionische organische Verbindungen mit 4 bis 14 C-Atomen, die eine oder mehrere polare Gruppen im Molekül enthalten.

Typische bekannte nichtaromatische Cotenside sind alphatische Alkohole wie Butanol, Pentanol, Hexanol, Octanol, Hexandiol oder Octandiol. Gemäß einer bevorzugten Ausführungsform der Erfindung werden als Cotenside n-Pentanol, n-Hexanol, 1,2-Hexandiol, 1,2-Heptandiol oder 1,2-Octandiol verwendet.

Weiterhin können als Cotenside bevorzugt auch Monoalkylether oder Monoalkylester auf Basis von Glycerin, Ethylenglykol, Propylenglykol oder Diethylenglykol mit Fettsäuren oder Alkoholen mit 6 bis 10 Kohlenstoffatomen verwendet werden.

Gemäß einer bevorzugten Ausführungsform der Erfindung werden als Cotenside aromatische Cotenside verwendet. Unter aromatischen Cotensiden im Sinne der vorliegenden Erfindung werden insbesondere grenzflächenaktive Substanzen verstanden, die eine oder mehrere Arylgruppen enthalten und die für sich alleine genommen in Wasser keine mizellaren Strukturen aufbauen.

Vorteilhafterweise zeichnen sich diese aromatischen Cotenside zusätzlich durch antimikrobielle Eigenschaften aus, d.h. es handelt es sich um aromatische Cotenside mit konservierenden Eigenschaften. Die Verwendung solcher Cotenside ermöglicht die Herstellung erfindungsgemäßer O/W-Emulsionen, die idealerweise ohne weitere Konservierungsmittel auskommen. Darüber hinaus ist der Zusatz weiterer üblicher Konservierungsmittel (als Hilfs- und Zusatzstoffe) natürlich möglich, wie sie etwa in DE102005011785.6 beschrieben sind.

Erfindungsgemäß besonders bevorzugt als aromatische Cotenside mit konservierenden Eigenschaften sind Phenoxyethanol und Benzylalkohol, alleine oder in Kombination mit einem oder mehreren Alkylparabenestern, vorzugsweise Methlyparaben, Ethylparaben, Propylparaben, Isopropylparaben, Butylparaben und/oder Isobutylparaben. Besonders bevorzugt ist die Verwendung von Mischungen von Alkylparabenestern und Phenoxyethanol, wie sie etwa unter den Handelsnamen Euxyl^{®} K 300 (Schülke & Mayr) oder Phenonip^{®} (Clariant) kommerziell erhältlich sind.

Wie erwähnt können auch Mischungen der genannten konservierungsaktiven aromatischen Cotenside mit anderen geeigneten Konservierungsmitteln eingesetzt werden. So kann beispielsweise auch eine Mischung aus Phenoxyethanol und Ethylhexylglycerin verwendet werden, wie sie kommerziell unter dem Namen Euxyl PE 9010 (Schülke & Mayr) erhältlich ist.

Unter Ölen im Sinne der vorliegenden Erfindung werden insbesondere Verbindungen ausgewählt aus der Gruppe Guerbetalkohole auf Basis von Fettalkoholen mit 6 bis 20, vorzugsweise 8 bis 10 Kohlenstoffatomen, Ester von linearen C1-C44-Fettsäuren mit linearen C1-C22- Fettalkoholen, Ester von verzweigten C1-C44-Carbonsäuren mit linearen C1-C22-Fettalkoholen, Ester von linearen C1-C44-Fettsäuren mit verzweigten Alkoholen, Ester von linearen und/oder verzweigten Fettsäuren mit mehrwertigen Alkoholen und/oder Guerbetalkoholen, Triglyceride auf Basis C1-C44 Fettsäuren, pflanzliche Öle, verzweigte primäre Alkohole, substituierte Cyclohexane, Guerbetcarbonate, Dialkyl(en)ether, Dialkyl (en)carbonate und/oder aliphatische bzw. naphthenische Kohlenwasserstoffe, Siliconöle, Dimethicone, Cyclomethicone, ethoxylierte und/oder propoxylierte organische Alkohole, ethoxylierte und/oder propoxylierte organische Säuren oder Mischungen daraus verstanden. Auch dem Fachmann bekannte Parfumöle können im Sinne der Erfindung als Ölphasen fungieren.

Gemäß einer bevorzugten Ausführungsform der Erfindung werden als Öle Esteröle, Öle auf Etherbasis, Kohlenwasserstoffe sowie propoxylierte organische Alkohole und Mischungen dieser Verbindungen eingesetzt.

Als Esteröle kommen insbesondere Mono- oder Diester von linearen und/oder verzweigten Mono- und/oder Dicarbonsäuren mit ≥2 bis ≤44 C-Atomen mit linearen und/oder verzweigten (insbesondere 2-Ethylhexanol), gesättigten oder ungesättigten Alkoholen mit ≥1 bis ≤22 C-Atomen in Betracht. Ebenso sind im erfindungsgemäßen Sinne die Veresterungsprodukte aliphatischer, difunktioneller oder trifunktioneller Alkohole (insbesondere Dimerdiol und/oder Trimerdiol) mit ≥2 bis ≤36 C-Atomen mit einer oder mehreren monofunktionellen aliphatischen Carbonsäuren mit ≥1 bis ≤22 C-Atomen geeignet. Weiterhin sind erfindungsgemäß auch Esteröle geeignet, die aromatische Gruppen enthalten.

Die teilweise Verwendung von Esterölen, die bei Raumtemperatur wachsartigen Charakter haben, wie etwa Myristyl Myristate, kann zu einem reichhaltigeren Hautgefühl der Emulsionen führen.

Als Etheröle kommen insbesondere Dialklylether mit ≥4 bis ≤24 C-Atomen in Betracht. Erfindungsgemäß bevorzugt geeignet sind gesättigte C6-C18-Dialkylether, wie beispielsweise Di-n-octylether, Di-(2-ethylhexyl)-ether, Laurylmethylether oder Octylbutylether, sowie Didodecylether.

Besonders bevorzugte Ölkomponenten sind die kosmetischen Esteröle Ethylhexylpalmitat, Ethylhexylstearat, Decylcocoat, Diethylhexylcarbonat, Dioctylcarbonat, Cetearylethylhexanoat, Decyloleat, Isocetylpalmitat, Cetearylisononanoat, Hexyllaurat, Isopropylisononaoat, Isopropylpalmitat, Isoproylmyristat, Isopropyllaurat und C12-15 Alkylbenzoat sowie das kosmetische Etheröl Dicaprylylether und die propoxylierten organischen Alkohole PPG15-Stearylether oder PPG-4 Butylether sowie Mischungen der genannten Verbindungen.

Unter "polaren Lösungsvermittlern" im Sinne der vorliegenden Erfindung werden insbesondere polare Verbindungen verstanden, die in Mengen von bis zu 10 Gew.-% den weiter unten beschriebenen Ölphasen beigesetzt werden, um klare Ölphasen zu erhalten. Vorzugsweise handelt es sich dabei um Wasser, Glykole, Polyalkylenglykole, Glycerin, Polyglycerin oder kurzkettige Alkohole wie Ethanol oder Isopropanol.

Als Hilfs- und Zusatzstoffe können alle dem Fachmann auf diesem Gebiet als Stand der Technik bekannten Hilfs- und Zusatzstoffe wie Öle und Wachse, handelsübliche Tenside oder Emulgatoren, Konsistenzgeber, Verdickungsmittel z.B. auf Polymerbasis, anorganische und organische UV-Lichtschutzfilter, Selbstbräuner, Pigmente, Antioxidantien, Hydrotrope, Deodorant- und Antitranspirantwirkstoffe, Wirkstoffe, Farbstoffe, zusätzliche Konservierungsmittel und Parfüms eingesetzt werden, wie sie etwa in DE102005011785.6 beschrieben sind.

Die Hilfs- und Zusatzstoffe können dabei sowohl der Öl- als auch der Wasserphase bzw. dem Verdünnungswasser im Herstellprozess der Emulsion zugesetzt werden.

Als Wirkstoffe sind dabei insbesondere Tocopherol, Tocopherolacetat, Tocopherolpalmitat, Ascorbinsäure, Desoxyribonucleinsäure, Coenzym Q10, Retinol- und Retinylderivate, Bisabolol, Allantoin, Phytantriol, Panthenol, AHA-Säuren, Aminosäuren, Hyaluronsäure, Creatin (und Creatinderivate), Guanidin (und Guanidinderivate), Ceramide, Phytosphingosin (und Phytosphingosinderivate), Sphingosin (und Sphingosinderivate), Pseudoceramide, essentielle Öle, Peptide, Proteinhydrolysate, Pflanzenextrakte und Vitaminkomplexe bevorzugt.

Die erfindungsgemäßen O/W-Emulsionen sind prinzipiell unter Benutzung eines einfachen Rührwerkzeugs herstellbar. Dabei wird kein zusätzlicher Homogenisierschritt benötigt.

Die Herstellung erfolgt bevorzugt bei Raumtemperatur durch direktes Eingießen einer klaren, einphasigen Ölphase, enthaltend eine Emulgatormischung, Cotenside, Öle und ggf. Übliche Hilfs- und Zusatzstoffe, ins Verdünnungswasser. Falls nötig, kann die Ölphase durch Zusatz von bis zu 10 Gew.-% eines polaren Lösungsvermittlers in eine klare Phase verwandelt werden. Solche polaren Lösungsvermittler können sein Wasser, Glykole, Polyalkylenglykole, Glycerin, Polyglycerine oder kurzkettige Alkohole wie Ethanol oder iso-Propanol. Bevorzugt wird als polarer Lösungsvermittler Wasser verwendet.

Homogene, klare Ölphasen sind zur Herstellung erfindungsgemäßer, feinteiliger O/W-Emulsionen vorteilhaft. Die Verwendung trüber Ölphasen führt in der Regel zu grobteiligeren Emulsionen, deren Langzeitstabilität oft unzureichend ist. Der Übergang von klaren zu trüben Ölphasen ist dabei fließend. Der Trübungsgrad, bei dem noch Emulsionen mit ausreichender Langzeitstabilität herstellbar sind, ist abhänging von Art und Menge der eingesetzten Komponenten und in diesen Grenzfällen individuell zu bestimmen.

Alternativ zur genannten Methode können erfindungsgemäße feinteilige Öl-in-Wasser-Emulsionen auch über die Zwischenstufe eines klaren bis transparenten mikroemulsionsartigen Konzentrats erfolgen. Dieses Konzentrat besteht in der Regel aus >30-≤90 Gew.-% Ölphase, bevorzugt aus ≥40-≤80 Gew.-% Ölphase, enthaltend eine Emulgatormischung, Cotenside, Öle und ggf. Polare Lösungsvermittler und/oder übliche Hilfs- und Zusatzstoffe. Diese klaren bis transparenten mikroemulsionsartigen Konzentrate werden vorzugsweise bei Raumtemperatur durch Einrühren von Wasser in die Ölphase hergestellt. Zur Herstellung dieser Konzentrate können auch trübe Ölphasen verwendet werden. Dabei ist der optimale Wassergehalt der Konzentrate rezepturabhängig (z.B. vom eingesetzten Öl), liegt aber in der Regel bei ≥10-<70 Gew.-%, bevorzugt bei ≥20-≤60 Gew.-%.

Diese mikroemulsionsartigen Konzentrate können schließlich zu erfindungsgemäßen Öl-in-Wasser-Emulsionen verdünnt werden. Dabei kann sowohl die Herstellung der mikroemulsionsartigen Konzentrate als auch der abschließende Verdünnungsschritt bei Raumtemperatur unter Verwendung eines einfachen Rührwerkzeugs erfolgen.

Ein weiterer Gegenstand der Erfindung sind daher Ölphasen enthaltend:
A) eine Emulgatormischung bestehend aus:
   a) mindestens einem nichtionischen primären Emulgator und
   b) mindestens einem ein oder mehrere Säurefunktionen enthaltenden sekundären Emulgator, dessen Säurefunktion gegebenenfalls ganz oder teilweise neutralisiert sein kann und
B) ein oder mehrere Cotenside und
C) ein oder mehrere Öle und
D) ≥0 bis < 10 Gew.-% (bezogen auf die gesamte Ölphase) eines oder mehrerer polarer Lösungsvermittler und gegebenfalls
E)übliche Hilfs- und Zusatzstoffe.

Erfindungsgemäß bevorzugt sind dabei homogene und klare Ölphasen.

Diese Ölphasen sind herstellbar nach den bekannten Verfahren des Standes der Technik. Beispielsweise können die Ölphasen je nach Konsistenz und Konzentration der verwendeten Komponenten bei Temperaturen im Bereich von ≥20 bis ≤75°C durch einfaches Vermischen der Komponenten hergestellt werden. Diese Ölphasen sind bei Raumtemperatur zur Herstellung der erfindungsgemäßen Öl-in-Wasser-Emulsionen verwendbar.

Ein weiterer Gegenstand der Erfindung ist ein Verfahren zur Herstellung erfindungsgemäßer Öl-in-Wasser-Emulsionen, welches dadurch gekennzeichnet ist, dass diese klaren Ölphasen vorzugsweise bei Temperaturen kleiner 40°C, insbesondere Raumtemperatur, mit einer entsprechenden Wasserphase unter an sich bekannten Bedingungen auf einen Gesamtwasserphasengehalt von ≥ 70 Gew.-%, bevorzugt ≥ 80 Gew.-%, eingestellt wird.

Ein weiterer Gegenstand der Erfindung sind klare bis transparente mikroemulsionsartige Konzentrate enthaltend:
A) eine Emulgatormischung bestehend aus:
   a) mindestens einem nichtionischen primären Emulgator und
   b)mindestens einem ein oder mehrere Säurefunktionen enthaltenden sekundären Emulgator, dessen Säurefunktion gegebenenfalls ganz oder teilweise neutralisiert sein kann und
B) ein oder mehrere Cotenside und
C) ein oder mehrere Öle und gegebenfalls
D) einen oder mehrere polare Lösungsvermittler und gegebenfalls
E)übliche Hilfs- und Zusatzstoffe,
mit der Maßgabe, dass der Gesamtwasserphasengehalt der mikroemulsionsartigen Konzentrate ≥10 bis <70 Gew.-%, bezogen auf das gesamte Konzentrat, beträgt.

Ein weiterer Gegenstand der Erfindung ist ein Verfahren zur Herstellung erfindungsgemäßer Öl-in-Wasser-Emulsionen, welches dadurch gekennzeichnet ist, dass diese mikroemulsionsartigen Konzentrate vorzugsweise bei Temperaturen kleiner 40°C, insbesondere Raumtemperatur, mit einer entsprechenden Wasserphase unter an sich bekannten Bedingungen auf einen Gesamtwasserphasengehalt von ≥ 70 Gew.-%, bevorzugt ≥ 80 Gew.-%, eingestellt wird.

Ein weiterer Gegenstand der Erfindung ist die Verwendung der erfindungsgemäßen Emulsionen zur Herstellung von kosmetischen, dermatologischen oder pharmazeutischen Zubereitungen. Insbesondere steht dabei der Einsatz als Tränklösungen zur Herstellung von Feuchttüchern, ganz besonders von kosmetischen Feuchttüchern zur Pflege und Reinigung der Haut im Vordergrund.

Ein weiterer Gegenstand der Erfindung ist die Verwendung der Emulsionen in kosmetischen Reinigungs- und Pflegezubereitungen für Haut- und Hautanhangsgebilde. Insbesondere steht dabei die Verwendung in sprühbaren Zubereitungen im Vordergrund, wie sie etwa für Gesichts- und Körperpflegeprodukte, Babypflege, Sonnenschutzpräparate, Make-up Remover sowie Antiperspirantien/Deodorantien verwendet werden.

Die erfindungsgemäßen Öl-in-Wasser-Emulsionen eignen sich auch hervorragend sowohl zur Herstellung von Reinigungs- und Pflegefeuchtüchern als auch zur direkten Anwendung in Form von sprühbaren Emulsionssystemen zur Reinigung und Pflege von Oberflächen in Haushalt und Industrie, wie etwa der Textilpflege, der Lederpflege, der Pflege und Reinigung von metallischen oder nichtmetallischen Oberflächen, beispielsweise zur Reinigung und Pflege von Automobilen oder Möbeln.

Ein weiterer Gegenstand der Erfindung ist demnach die Verwendung der Emulsionen zur Herstellung von Reinigungs- und Pflegemitteln für Haushalt und Industrie, wie Textilien, Leder, Kunststoffe, metallische und nichtmetallische Oberflächen. Insbesondere steht dabei der Einsatz als Tränklösungen zur Herstellung von Feuchttüchern sowie die Verwendung in sprühbaren Zubereitungen im Vordergrund.

Die hier beschriebene technische Lehre ermöglicht auf einfache Weise bei Raumtemperatur, PEG-freie, niedrigviskose, feinteilige und langzeitstabile Öl-in-Wasser-Emulsionen herzustellen, die bereits eine ausreichende Konservierung aufweisen.

### Ausführungsbeispiele:

Die folgenden Beispiele sollen den Gegenstand der Erfindung näher erläutern, ohne ihn auf diese Beispiele einzuschränken.

Die Konzentrationsangaben in allen Beispielen sind als Gew.-% angegeben.

Die Herstellung der erfindungsgemäßen Ölphasen, der erfindungsgemäßen mikroemulsionsartigen Konzentrate und der erfindungsgemäßen Emulsionen erfolgte unter Verwendung eines einfachen manuellen Rührwerkzeugs. Nach Herstellung der entsprechenden Ölphasen erfolgte sowohl die Überführung in erfindungsgemäße mikroemulsionsartige Konzentrate und in erfindungsgemäße Öl-in-Wasser-Emulsionen bei Temperaturen < 30°C.

Bei der Herstellung der Emulsionen ist es dabei vorteilhaft, die als Teil der Emulgatormischung eingesetzten Säurepartialester (b) von Anfang an zumindest in teilneutralisierter Form einzusetzen. Allerdings ist es auch möglich, diesen Neutralisationsschritt zu einem späteren Punkt des Herstellprozesses durchzuführen und zunächst mit den nichtneutralisierten Säurepartialestern zu arbeiten.

In den Beispielen wurden die eingesetzten Säurepartialester in neutralisierter Form eingesetzt.

### Beispielemulgatoren 1-11:

Beschreibung der in den Beispielformulierungen eingesetzten Emulgatorsysteme (die Gesamt(gewichts)prozentzahl pro Emulgatorsystem addiert sich jeweils auf 100):

| Emulgator 1: | |
|---|---|
| Emulgatorkomponente A: | 97% Polyglyceryl-4 Laurate¹⁾ |
| Emulgatorkomponente B: | 3% Dilauryl Citrate²⁾ |

| Emulgator 2: | |
|---|---|
| Emulgatorkomponente A: | 96% Polyglyceryl-4 Laurate¹⁾ |
| Emulgatorkomponente B: | 4% Dilauryl Citrate²⁾ |

| Emulgator 3: | |
|---|---|
| Emulgatorkomponente A: | 95% Polyglyceryl-4 Laurate¹⁾ |
| Emulgatorkomponente B: | 5% Dilauryl Citrate²⁾ |

| Emulgator 4: | |
|---|---|
| Emulgatorkomponente A: | 92% Polyglyceryl-4 Laurate¹⁾ |
| Emulgatorkomponente B: | 8% Dilauryl Citrate²⁾ |

| Emulgator 5: | |
|---|---|
| Emulgatorkomponente A: | 98.5% Polyglyceryl-4 Laurate¹⁾ |
| Emulgatorkomponente B: | 1.5% Disodium Lauryl Sulfosuccinate³ |

| Emulgator 6: | |
|---|---|
| Emulgatorkomponente A: | 98.5% Polyglyceryl-3 Laurate⁴⁾ |
| Emulgatorkomponente B: | 1.5% Potassium Cetyl Phosphate⁵⁾ |

| Emulgator 7: | |
|---|---|
| Emulgatorkomponente A: | 77% Polyglyceryl-4 Laurate¹⁾ |
| | 19% Sorbitan Laurate⁶⁾ |
| Emulgatorkomponente B: | 4% Dilauryl Citrate²⁾ |

| Emulgator 8: | |
|---|---|
| Emulgatorkomponente A: | 64% Polyglyceryl-6 Laurate⁷⁾ |
| | 29% Polyglyceryl-4 Laurate¹⁾ |
| Emulgatorkomponente B: | 7% Dilauryl Citrate²⁾ |

| Emulgator 9: | |
|---|---|
| Emulgatorkomponente A: | 49% Polyglyceryl-6 Laurate⁷⁾ |
| | 46% Sorbitan Laurate⁶⁾ |
| Emulgatorkomponente B: | 5% Dilauryl Citrate²⁾ |

| Emulgator 10: | |
|---|---|
| Emulgatorkomponente A: | 19% Polyglyceryl-10 Laurate⁸⁾ |
| | 77% Sorbitan Laurate⁶⁾ |
| Emulgatorkomponente B: | 4% Dilauryl Citrate²⁾ |

| Emulgator 11: | |
|---|---|
| Emulgatorkomponente A: | 94% Polyglyceryl-4 Laurate¹⁾ |
| Emulgatorkomponente B: | 6% Dilauryl Citrate²⁾ |

| | |
|---|---|
| 1) TEGO^{®} Care PL 4 (Degussa) 2) Mit KOH teilneutralisierter Diester aus Laurylalkohol und Zitronensäure 3) REWOFOL^{®} SB F 12 P (Degussa) 4) Polyglyceryl-3 Laurate mit Veresterungsgrad von 20% der OH-Gruppen 5) Amphisol^{®} K (Roche) 6) TEGO^{®} SML (Degussa) 7) Polyglyceryl-6 Laurate mit Veresterungsgrad von 13% der OH-Gruppen 8) Polyglyceryl-10 Laurate mit Veresterungsgrad von 8% der OH-Gruppen | |

### Beispiele für klare Ölphasen 1-10:

Diese Beispiele sollen insbesondere zeigen, wie sich erfindungsgemäße klare Ölphasen zusammensetzen können (Angaben in Gew.-%), die in einem weiteren Schritt bei Raumtemperatur zu erfindungsgemäßen, feinteiligen Öl-in-Wasser Emulsionen verarbeitet werden können (siehe Beispielemulsionen 1-10).

| Klare Ölphasen | 1 | 2 | 3 | 4 | 5 |
|---|---|---|---|---|---|
| Emulgator 2 | | 22,0 % | 20,8 % | | |
| Emulgator 3 | 22,0 % | | | 22,0 % | |
| Emulgator 5 | | | | | 22,0 % |
| Isopropyl Palmitate | 66,0 % | | | 66,0 % | 66,0 % |
| Ethylhexyl Palmitate | | | 62,5 % | | |
| Diethylhexyl Carbonate | | 66,0 % | | | |
| Euxyl^{®} K 300⁹⁾ | 12,0 % | 12,0 % | 11,7 % | | 12,0 % |
| Benzyl Alcohol | | | | 12,0 % | |
| Wasser | | | 5,0 % | | |

| Klare Ölphasen | 6 | 7 | 8 | 9 | 10 |
|---|---|---|---|---|---|
| Emulgator 5 | | | | | |
| Emulgator 7 | | 23,6 % | 22,9 % | | |
| Emulgator 8 | 21,7 % | | | | |
| Emulgator 11 | | | | 23,4 % | 23,5 % |
| Sorbitan Octanoate¹⁰⁾ | | | | 1,7 % | 2,8 % |
| Isopropyl Palmitate | | | | 35,1 % | 35,0 % |
| Ethylhexyl Palmitate | | 70,7 % | 68,7 % | | |
| C12-15 Alkyl Benzoate | 65,1 % | | | | |
| Isohexadecane | | | | 35,1 % | |
| Paraffinöl (25 mPas bei 30°C) | | | | | 35,0 % |
| Phenoxyethanol | | | | | 3,7 % |
| Euxyl^{®} K 300⁹⁾ | 12,2 % | | 7,4 % | 4,7 % | |
| Euxyl^{®} PE 9010¹¹⁾ | | 5,7 % | | | |
| Wasser | 1,0 % | | 1,0 % | | |

| | | | | | |
|---|---|---|---|---|---|
| ⁹⁾Euxyl^{®} K 300(Schülke & Mayr): Phenoxyethanol, Methylparaben, Ethylparaben, Propylparaben, Butylparaben, Isopropylparaben ¹⁰⁾TEGOTENS^{®} SD (Degussa) ¹¹⁾ Euxyl^{®} PE 9010 (Schülke & Mayr): Phenoxyethanol, Ethylhexylglycerin | | | | | |

### Beispiele für klare bis transparente mikroemulsionsartige Konzentrate 1-10:

Diese Beispiele sollen insbesondere zeigen, wie sich erfindungsgemäße klare bis transparente mikroemulsionsartige Konzentrate zusammensetzen können(Angaben in Gew.-%), die in einem weiteren Schritt bei Raumtemperatur zu erfindungsgemäßen, feinteiligen Öl-in-Wasser-Emulsionen verarbeitet werden können (siehe Beispielemulsionen 11-20).

| Mikroemulsionsartige Konzentrate | 1 | 2 | 3 | 4 | 5 |
|---|---|---|---|---|---|
| Emulgator 1 | 14,3 % | 13,2 % | | | |
| Emulgator 4 | | | | 9,1 % | |
| Emulgator 6 | | | 8,8 % | | |
| Emulgator 7 | | | | | 12,5 % |
| Decyl Cocoate | | 39,6 % | | | |
| Ethylhexyl Palmitate | 42,9 % | | 26,4 % | 27,4 % | 37,5 % |
| Euxyl^{®} K 300⁹⁾ | 7,8 % | 7,2 % | 4,8 % | 8,5 % | |
| Phenoxyethanol | | | | | 5,0 % |
| Wasser | 35,0 % | 40,0 % | 60,0 % | 55,0 % | 45,0 % |

| Mikroemulsionsartige Konzentrate | 6 | 7 | 8 | 9 | 10 |
|---|---|---|---|---|---|
| Emulgator 2 | | | | | 14,3 % |
| Emulgator 7 | 13,2 % | | | | |
| Emulgator 9 | | 14,3 % | | | |
| Emulgator 10 | | | 18,6 % | | |
| Emulgator 11 | | | | 14,3 % | |
| Diethylhexyl Carbonate | | | | 42,8 % | 32,2 % |
| Ethylhexyl Palmitate | | 42,9 % | 55,9 % | | |
| Cetearyl Isononanoate | 39,6 % | | | | |
| Cyclopentasiloxane | | | | | 10,7 % |
| Euxyl^{®} K 300⁹⁾ | | 7,8 % | 10,5 % | | |
| Phenoxyethanol | | | | | 7,8 % |
| Benzyl Alcohol | 7,2 % | | | | |
| Capryl Glycol¹²⁾ | | | | 2,9% | |
| Wasser | 40,0 % | 35,0 % | 15,0 % | 40,0% | 35,0 % |

| | | | | | |
|---|---|---|---|---|---|
| ¹²⁾Dermosoft^{®} Octiol (Dr. Straetmans) (1,2-Octandiol) | | | | | |

### Beispielemulsionen 1-24:

Die Emulsionen 1-24 sollen den Aufbau erfindungsgemäßer Emulsionen beispielhaft veranschaulichen(Angaben in Gew.-%).

Dabei wurden die Emulsionen 1-10 durch Eingießen der klaren Ölphasen (Beispiele 1-10 (s.o.)) in Wasser bei Raumtemperatur unter Verwendung eines einfachen manuellen Rührwerkzeugs hergestellt.

Die Emulsionen 11-20 wurden ebenfalls bei Raumtemperatur durch Verdünnen der klaren bis transparenten mikroemulsionsartigen Konzentrate (entsprechende Beispiele 1-10 (s.o.)) unter Verwendung eines einfachen manuellen Rührwerkzeugs hergestellt.

Die Beispielemulsionen 21-24 basieren auf den klaren Ölphasen 2 und 3 und wurden daraus durch Verdünnen unter Durchmischung hergestellt. Dabei wurden der Wasserphase exemplarisch wasserlösliche Hilfs- und Zusatzstoffe beigesetzt.

Bei einigen Beispielen wurde erfindungsgemäß Parfum als Hilfs -und Zusatzstoff zugesetzt, das ohne weiteres in den entsprechenden Mengen in den klaren Ölphasen bzw. in klaren bis transparenten mikroemulsionsartigen Konzentraten gelöst werden konnte.

Alle Beispielemulsionen sind niedrigviskos, feinteilig und langzeitstabil.

| Beispielemulsionen | 1 | 2 | 3 | 4 | 5 |
|---|---|---|---|---|---|
| Emulgator 2 | | 1,25 % | 1,25 % | | |
| Emulgator 3 | 1,25 % | | | 1,25 % | |
| Emulgator 5 | | | | | 1,25 % |
| Isopropyl Palmitate | 3,75 % | | | 3,75 % | 3,75 % |
| Ethylhexyl Palmitate | | | 3,75 % | | |
| Diethylhexyl Carbonate | | 3,75 % | | | |
| Euxyl^{®} K 300⁹⁾ | 0,7 % | 0,7 % | 0,7 % | | 0,7 % |
| Benzyl Alcohol | | | | 0,7% | |
| Parfum | | | | | 0,2 % |
| Wasser | 94,3 % | 94,3 % | 94,3 % | 94,3 % | 94,1% |

| Beispielemulsionen | 6 | 7 | 8 | 9 | 10 |
|---|---|---|---|---|---|
| Emulgator 7 | | 1,25 % | 2,5 % | | |
| Emulgator 8 | 1,25 % | | | | |
| Emulgator 11 | | | | 4,0 % | 5,0 % |
| Sorbitan Octanoate¹⁰⁾ | | | | 0,3 % | 0,6 % |
| Isopropyl Palmitate | | | | 6,0 % | 7,5 % |
| Ethylhexyl Palmitate | | 3,75 % | 7,5 % | | |
| C12-15 AlkylBenzoate | 3,75 % | | | | |
| Isohexadecane | | | | 6,0 % | |
| Paraffinöl (25 mPas bei 30°C) | | | | | 7,5 % |
| Phenoxyethanol | | | | | 0,8 % |
| Euxyl^{®} K 300⁹⁾ | 0,7 % | | 0,8 % | 0,8 % | |
| Euxyl^{®} PE 9010¹¹⁾ | | 0,3 % | | | |
| Parfum | 0,2 % | 0,2 % | 0,2 % | | |
| Wasser (total) | 94,1 % | 94,5 % | 89,0 % | 82,9 % | 78,6 % |

| Beispielemulsionen | 11 | 12 | 13 | 14 | 15 |
|---|---|---|---|---|---|
| Emulgator 1 | 1,25 % | 1,25 % | | | |
| Emulgator 4 | | | | 0,75 % | |
| Emulgator 6 | | | 1,25 % | | |
| Emulgator 7 | | | | | 1,25 % |
| Decyl Cocoate | | 3,75 % | | | |
| Ethylhexyl Palmitate | 3,75 % | | 3,75 % | 2,25 % | 3,75 % |
| Phenoxyethanol | | | | | 0,5 % |
| Euxyl^{®} K 300⁹⁾ | 0,7 % | 0,7 % | 0,7 % | 0,7 % | |
| Parfum | 0,2 % | 0,2 % | 0,2 % | | |
| Wasser (total) | 94,1 % | 94,1 % | 94,1 % | 96,3 % | 94,3 % |

| Beispielemulsionen | 16 | 17 | 18 | 19 | 20 |
|---|---|---|---|---|---|
| Emulgator 2 | | | | | 1,25 % |
| Emulgator 7 | 1,25 % | | | | |
| Emulgator 9 | | 1,25 % | | | |
| Emulgator 10 | | | 1,25 % | | |
| Emulgator 11 | | | | 2,5 % | |
| Diethylhexyl Carbonate | | | | 7,5 % | 2,81 % |
| Ethylhexyl Palmitate | | 3,75 % | 3,75 % | | |
| Cetearyl Isononanoate | 3,75 % | | | | |
| Cyclopentasiloxane | | | | | 0,94 % |
| Euxyl^{®} K 300⁹⁾ | | 0,7 % | 0,7 % | | |
| Phenoxyethanol | | | | | 0,7 % |
| Benzyl Alcohol | 0,7 % | | | | |
| Capryl Glycol¹²⁾ | | | | 0,5 % | |
| Parfum | 0,2 % | 0,2 % | 0,2 % | | |
| Wasser (total) | 94,1 % | 94,1 % | 94,1 % | 89,5 % | 94,3 % |

| Beispielemulsionen | 21 | 22 | 23 | 24 |
|---|---|---|---|---|
| Emulgator 2 | 1,25 % | 1,25 % | 1,25 % | 1,25 % |
| Ethylhexyl Palmitate | 3,75 % | | 3,75 % | |
| Diethylhexyl Carbonate | | 3,75 % | | 3,75 % |
| Euxyl^{®} K 300⁹⁾ | 0,7 % | 0,7 % | 0,7 % | 0,7 % |
| Ethanol | 3,0 % | | | |
| Glycerin | | 3,0 % | | |
| Panthenol | | | 0,5 % | |
| Creatine | | | | 0,25 % |
| Wasser (total) | 91,3 % | 91,3 % | 93,8 % | 94,05 % |

Partikelgrößenbestimmung durch Dynamische Lichtstreuung:

Beispielhaft für den extrem feinen Dispersionsgrad der erfindungsgemäßen Emulsionen wurde mit Hilfe von Dynamischer Lichtstreuung die Partikelgröße einzelner Beispielemulsionen charakterisiert.

Dynamische Lichtstreuung beruht auf der Analyse der Schwankungen der Streulichtintensität diffundierender Partikel in Lösung. Für verdünnte Lösungen kann dadurch der Diffusionskoeffizient der Teilchen in Lösung bestimmt werden, der über die Stokes-Einstein-Gleichung in einen mittleren hydrodynamischen Radius <rₕ> der Teilchen (hier: Emulsionströpfchen) umgerechnet werden kann.

Im vorliegenden Fall wurden die genannten Beispielemulsionen mit vollentsalztem Wasser um einen Faktor 10 verdünnt und mit Hilfe eines Dynamischen Lichtstreugeräts der Firma Malvern Instruments (HPPS 3.1) bei 25°C charakterisiert. Angegeben sind jeweils die Mittelwerte aus jeweils drei Messungen mit jeweils 100 Sekunden Messzeit.

| Beispielemulsion | <rₕ> in nm |
|---|---|
| 2 | 55 |
| 3 | 60 |
| 6 | 75 |
| 8 | 75 |
| 18 | 110 |
| 20 | 45 |

## Patentansprüche

1. Langzeitstabile, niedrigviskose, feinteilige Öl-in-Wasser-Emulsionen, enthaltend:
A) eine Emulgatormischung bestehend aus:
a) mindestens einem nichtionischen primären Emulgator und
b) mindestens einem, ein oder mehrere Säurefunktionen enthaltenden, sekundären Emulgator, dessen Säurefunktion gegebenenfalls ganz oder teilweise neutralisiert sein kann und
B) ein oder mehrere Cotenside und
C) ein oder mehrere Öle und gegebenenfalls
D) ein oder mehrere polare Lösungsvermittler und gegebenfalls
E) übliche Hilfs- und Zusatzstoffe,
mit der Maßgabe, dass der Wasserphasengehalt der Emulsionen ≥ 70 Gew.-%, bezogen auf Gesamtemulsion, liegt.

2. Eine Öl-in-Wasser-Emulsion nach Anspruch 1, **dadurch gekennzeichnet, dass** sie einen Wasserphasengehalt von ≥80-≤99 Gew.-% aufweist.

3. Eine Öl-in-Wasser-Emulsion nach Anspruch 1 oder 2, **dadurch gekennzeichnet, dass** Emulgatormischung (A), Cotenside (B) und Öle (C) in Gewichtsanteilen von ≥10-s30 (A), ≥3-≤20 (B) und ≥50-≤87 (C) vorliegen.

4. Eine Öl-in-Wasser-Emulsion nach einem oder mehreren der Ansprüche 1 bis 3, **dadurch gekennzeichnet, dass** sich die Emulgatormischung (A) zusammensetzt aus ≥75-≤99,5 Gew.-% nichtionischer primärer Emulgatorkomponente(a) und ≥0,5-≤25 Gew.-% Säuregruppen enthaltender Emulgatorkomponente (b).

5. Eine Öl-in-Wasser-Emulsion nach einem oder mehreren der Ansprüche 1 bis 4, **dadurch gekennzeichnet, dass** in der Emulgatormischung (A) die primäre nichtionische Emulgatorkomponente (a) einen oder mehrere Emulgatoren enthält, ausgewählt aus folgenden Gruppen:
a1) Glycerin- und Polyglycerinpartialester, vorzugsweise hergestellt durch Veresterung von aliphatischen, linearen oder verzweigten, ggf. ungesättigten und/oder hydroxyfunktionalisierten Carbonsäuren mit einer Kettenlänge von ≥6 bis ≤ 22 C-Atomen mit Glycerin, Polyglycerinen oder Gemischen aus beiden,
a2) Sorbitan oder Sorbitolpartialester, vorzugsweise hergestellt durch Veresterung von aliphatischen, linearen oder verzweigten, ggf. ungesättigten und/oder hydroxyfunktionalisierten Carbonsäuren mit einer Kettenlänge von ≥6 bis ≤22 C-Atomen mit Sorbitol,
a3) Kohlenhydratester, bevorzugt Glykosid- oder Sucroseester, vorzugsweise hergestellt durch Veresterung von aliphatischen, linearen oder verzweigten, ggf. ungesättigten und/oder hydroxyfunktionalisierten Carbonsäuren mit einer Kettenlänge von ≥6 bis ≤22 C-Atomen mit Mono- oder Polysacchariden, sowie gegebenenfalls darüber hinaus,
a4) (Alkylpoly)Glykoside, vorzugsweise hergestellt durch Umsetzung von aliphatischen, linearen oder verzweigten, ggf. ungesättigten und/oder zusätzlich hydroxyfunktionalisierten Alkoholen mit einer Kettenlänge von ≥6 bis ≤22 C-Atomen mit Mono- oder Polysacchariden
oder Mischungen daraus.

6. Eine Öl-in-Wasser-Emulsion nach einem oder mehreren der Ansprüche 1 bis 5, **dadurch gekennzeichnet, dass** die primäre nichtionische Emulgatorkomponente (a) aus einer Mischung von ≥25-≤100 Gew.-% Polyglycerinpartialestern und ≥0-≤75 Gew.-% Sorbitanestern besteht, die als hydrophobe Anteile Fettsäurereste mit einer Kettenlänge von ≥10 bis ≤18 C-Atomen enthalten.

7. Eine Öl-in-Wasser-Emulsion nach einem oder mehreren der Ansprüche 1 bis 6, **dadurch gekennzeichnet, dass** die primäre nichtionische Emulgatorkomponente (a) aus Polyglycerinlauraten oder Mischungen von PolyglycerinLauraten mit Sorbitanlauraten besteht.

8. Eine Öl-in-Wasser-Emulsion nach einem oder mehreren der Ansprüche 1 bis 7, **dadurch gekennzeichnet, dass** die - ein oder mehrere Säurefunktionen enthaltende - Emulgatorkomponente (b) einen oder mehrere Emulgatoren enthält, die ausgewählt sind aus mindestens einer der Gruppen:
b1) Gegebenenfalls Hydroxylgruppen enthaltenden Di- oder Polycarboxylaten, sulfatierten, sulfonierten oder phosphatierten Carboxylaten, Malonaten, Malaten, Succinaten, Sulfosuccinaten, Citraten, Tartraten, bei denen die Säuregruppen teilweise verestert wurden mit aliphatischen oder aromatischen, linearen oder verzweigten, ggf. ungesättigten und/oder hydroxyfunktionalisierten Alkoholen mit einer Kettenlänge von ≥6 bis ≤22 C-Atomen,
b2) Gegebenenfalls Hydroxylgruppen enthaltenden Di- oder Polycarboxylaten, sulfatierten oder sulfonierten oder phosphatierten Carboxylaten, Malonaten, Malaten, Succinaten, Sulfosuccinaten, Citraten, Tartraten, bei denen die Säuregruppen teilweise verestert wurden mit Polyolen, Polyolpartialestern, bevorzugt aus Glycerin, Polyglycerin und/oder Sorbitol mit aliphatischen oder aromatischen, linearen oder verzweigten, ggf. ungesättigten und/oder hydroxyfunktionalisierten Carbonsäuren mit einer Kettenlänge von ≥6 bis ≤22 C-Atomen,
b3) Polyolen, bevorzugt Glycerin, Polyglycerin und Sorbitol, welche teilverestert sind mit aliphatischen oder aromatischen, linearen oder verzweigten, ggf. ungesättigten und/oder hydroxyfunktionalisierten Mono-, Di- oder Polycarbonsäuren mit einer Kettenlänge von ≥2 bis ≤ 22 C-Atomen, mit der Maßgabe, dass im Molkekül freie, neutralisierbare Säuregruppen vorliegen,
b4) Hydroxyfunktionellen Mono-, Di- oder Polycarbonsäuren, deren Hydroxylgruppen zumindest partiell mit aliphatischen, linearen oder verzweigten, ggf. ungesättigten und/oder hydroxyfunktionalisierten Carbonsäuren mit einer Kettenlänge von ≥6 bis ≤22 C-Atomen umgesetzt wurden,
b5) N-Acylaminosäuren, wie Sarcosinaten, Glutamaten, Asparaginaten, enthaltend einen aliphatischen oder aromatischen, linearen oder verzweigten, ggf. ungesättigten und/oder hydroxyfunktionalisierten Rest mit einer Kettenlänge von ≥6 bis ≤22 C-Atomen,
b6) Carboxylaten, Sulfaten, Sulfonaten, Phosphonaten oder Phosphaten, enthaltend einen aliphatischen oder aromatischen, linearen oder verzweigten, ggf. ungesättigten und/oder hydroxyfunktionalisierten Rest mit einer Kettenlänge von ≥6 bis ≤ 22 C-Atomen
oder Mischungen daraus.

9. Eine Öl-in-Wasser-Emulsion nach mindestens einem der Ansprüche 1 bis 8, **dadurch gekennzeichnet, dass** als sekundäre Emulgatorkomponente b) teilneutralisierte oder neutralisierte Zitronensäurepartialester verwendet werden, deren hydrophobe Reste jeweils ≥10 bis ≤18 C-Atome enthalten.

10. Eine Öl-in-Wasser-Emulsion nach mindestens einem der Ansprüche 1 bis 9, **dadurch gekennzeichnet, dass** mindestens ein aliphatisches Cotensid (B) eingesetzt wird, ausgewählt aus der Gruppe n-Pentanol, n-Hexanol, 1,2-Hexandiol, 1,2-Heptandiol und 1,2-Octandiol.

11. Eine Öl-in-Wasser-Emulsion nach mindestens einem der Ansprüche 1 bis 10, **dadurch gekennzeichnet, dass** mindestens ein aromatisches Cotensid (B) eingesetzt wird.

12. Eine Öl-in-Wasser-Emulsion nach mindestens einem der Ansprüche 1 bis 11, **dadurch gekennzeichnet, dass** als aromatische Cotenside (B) Phenoxyethanol, Benzylalkohol, Alkylparabenester alleine oder in Mischungen miteinander und/oder üblichen Konservierungsmitteln eingesetzt werden.

13. Eine Öl-in-Wasser-Emulsion nach mindestens einem der Ansprüche 1 bis 12, **dadurch gekennzeichnet, dass** als Öle (C) kosmetische Ester- oder Etheröle eingesetzt werden.

14. Eine Öl-in-Wasser-Emulsion nach mindestens einem der Ansprüche 1 bis 13,**dadurch gekennzeichnet, dass** als Öl mindestens eine Verbindung eingesetzt wird, ausgesucht aus der Gruppe: Ethylhexylpalmitat, Ethylhexylstearat, Decylcocoat, Diethylhexylcarbonat, Dioctylcarbonat, Cetearylethylhexanoat, Decyloleat, Isocetylpalmitat, Cetearylisononanoat, Hexyllaurat, Isopropylisononaoat, Isopropylstearat, Isopropylpalmitat, Isoproylmyristat, Isopropyllaurat, C12-15 Alkylbenzoat, Dicaprylyl Ether, Mineralöl, Isohexadecan, Cyclopentasiloxan, Octyldodecanol oder Mischungen dieser Verbindungen.

15. Ölphasen enthaltend:
A) eine Emulgatormischung bestehend aus:
a) mindestens einem nichtionischen primären Emulgator und
b) mindestens einem, ein oder mehrere Säurefunktionen enthaltenden, sekundären Emulgator, dessen Säurefunktion gegebenenfalls ganz oder teilweise neutralisiert sein kann und
B) ein oder mehrere Cotenside und
C) ein oder mehrere Öle und
D) ≥0 bis < 10 Gew.-%, bezogen auf die gesamte Ölphase, eines oder mehrerer polarer Lösungsvermittler und gegebenfalls
E) übliche Hilfs- und Zusatzstoffe.

16. Ölphasen gemäß Anspruch 15, **dadurch gekennzeichnet, dass** sie homogen und klar sind.

17. Verfahren zur Herstellung von Öl-in-Wasser-Emulsionen, **dadurch gekennzeichnet, dass** Ölphasen nach Anspruch 15 oder 16 mit einer entsprechenden Wasserphase unter an sich bekannten Bedingungen auf einen Gesamtwasserphasengehalt von ≥ 70 Gew.-% eingestellt werden.

18. Klare bis transparente mikroemulsionsartige Konzentrate enthaltend:
A) eine Emulgatormischung bestehend aus:
a) mindestens einem nichtionischen primären Emulgator und
b) mindestens einem, ein oder mehrere Säurefunktionen enthaltenden, sekundären Emulgator, dessen Säurefunktion gegebenenfalls ganz oder teilweise neutralisiert sein kann und
B) ein oder mehrere Cotenside und
C) ein oder mehrere Öle und gegebenfalls
D) einen oder mehrere polare Lösungsvermittler und gegebenfalls
E) übliche Hilfs- und Zusatzstoffe,
mit der Maßgabe, dass der Gesamtwasserphasengehalt der mikroemulsionsartigen Konzentrate ≥10 bis <70 Gew.-%, bezogen auf das gesamte Konzentrat, beträgt.

19. Verfahren zur Herstellung von Öl-in-Wasser-Emulsionen, **dadurch gekennzeichnet, dass** klare bis transparente mikroemulsionsartige Konzentrate nach Anspruch 18 mit einer entsprechenden Wasserphase unter an sich bekannten Bedingungen auf einen Gesamtwasserphasengehalt von ≥ 70 Gew.-% eingestellt werden.

20. Verwendung der Öl-in-Wasser-Emulsionen gemäß mindestens einem der Ansprüche 1 bis 14 zur Herstellung von kosmetischen, dermatologischen oder pharmazeutischen Zubereitungen.

21. Verwendung von Öl-in-Wasser-Emulsionen gemäß Anspruch 20 zur Herstellung von Tränktüchern oder in sprühbaren Zubereitungen für Gesichts- und Körperpflege, Babypflege, Sonnenschutz, Make-Up Remover, Antiperspirantien/Deodorantien.

22. Verwendung von Öl-in-Wasser-Emulsionen nach einem der Ansprüche 1 bis 14 zur Herstellung von Reinigungs- und Pflegemitteln für Haushalt und Industrie.

23. Verwendung von Öl-in-Wasser-Emulsionen gemäß Anspruch 22 zur Herstellung von Tränktüchern oder in sprühbaren Zubereitungen zur Reinigung und Pflege von Textilien, Leder, Kunststoffen, metallischen und nichtmetallischen Oberflächen.
